# EUROPEAN PATENT APPLICATION

(11) **EP 3 360 581 A1**
(43) Date of publication of application: **15.08.2018**
(21) Application number: 16853944.3
(22) Date of filing: 07.10.2016
(51) Int. Cl.: A61K 48/00, A61K 39/395

(54) **PHARMACEUTICAL COMPOSITION FOR PREVENTING OR TREATING CANCER COMPRISING INHIBITOR OF PLRG1 (PLEIOTROPIC REGULATOR 1) AS ACTIVE INGREDIENT**

(30) Priority: 08.10.2015 KR 20150141667
(71) Applicant: Korea Institute of Radiological & Medical Sciences, Seoul 01812 (KR)
(72) Inventor: LEE, Kee-Ho, Seoul 01374 (KR); KIM, Sungsub, Daejeon 34049 (KR); KIM, Yeon-Soo, Daejeon 34178 (KR); PARK, Eun-Ran, Seoul 01813 (KR); SHIN, Hyun Jin, Seoul 05348 (KR); LEE, Eun-Ju, Seoul 05577 (KR); HAM, Yong-Ho, Seoul 05577 (KR); KIM, Sang Bum, Seoul 04424 (KR); PARK, Sun-Hoo, Seoul 04983 (KR); HAN, Chul-Ju, Seoul 04558 (KR); HONG, Sung Hee, Seoul 01318 (KR); KIM, Yang Hyun, Seoul 01843 (KR); KIM, Jung Min, Seoul 03410 (KR); KIM, Mi Yeun, Incheon 21377 (KR); KANG, Moonkyoung, Daejeon 34120 (KR); SONG, Eun Yeong, Cheongju-si Chungcheongbuk-do 28748 (KR); SONG, Jie Young, Seoul 07324 (KR)
(74) Representative: Gassner, Birgitta
(86) International application number: PCT/KR2016/011272
(87) International publication number: WO 2017/061828

(57) **Abstract**

The present invention relates to a pharmaceutical composition for preventing or treating cancer containing pleiotropic regulator 1 (PLRG1) inhibitor as an active ingredient, a method for treating cancer including administering the composition to a subject, a composition for diagnosing cancer containing an agent for measuring the expression level of PLRG1, a method for providing information for diagnosing cancer including measuring the expression level of PLRG1, and a method for screening agents for preventing or treating cancer.

Pleiotropic regulator 1 (PLRG1) is overexpressed in cancer cells, and the inhibition of the expression of PLRG1 can induce cancer cell-specific apoptosis. Accordingly, the PLRG1 inhibitor of the present invention has an excellent effect as an anticancer agent without side effects, and additionally, the PLRG1 inhibitor can be used for cancer diagnosis, screening of anticancer agents, *etc.* by measuring the expression levels of PLRG1.

<110>     KOREA INSTITUTE OF RADIOLOGICAL & MEDICAL SCIENCES

<120>     A composition for treating cancer comprising an inhibitor of
          PLRG1

<130>     OPA16253-PCT

<150>     KR 10-2015-0141667
<151>     2015-10-08

<160>     26

<170>     KoPatentIn 3.0

<210>     1
<211>     21
<212>     DNA
<213>     Artificial Sequence

<220>
<223>     PLRG1 RT-PCT primer-F


<400>     1
accgtccaca gcctacagcg a                                                  21


<210>     2
<211>     20
<212>     DNA
<213>     Artificial Sequence

<220>
<223>     PLRG1 RT-PCT primer-R


<400>     2
actatcacgc cccgcacagt                                                    20


<210>     3
<211>     20
<212>     DNA
<213>     Artificial Sequence

<220>
<223>     GAPDH RT-PCT primer-F


<400>     3
gtcagtggtg gacctgacct                                                    20


<210>     4
<211>     20
<212>     DNA
<213>     Artificial Sequence

<220>
<223>     GAPDH RT-PCT primer-R


<400>     4

tgctgtagcc aaattcgttg                                                    20

<210>  5
<211>  20
<212>  DNA
<213>  Artificial Sequence

<220>
<223>  b-actin RT-PCT primer-F

<400>  5
ggacttcgag caagagatgg                                                    20

<210>  6
<211>  20
<212>  DNA
<213>  Artificial Sequence

<220>
<223>  b-actin RT-PCT primer-R

<400>  6
agcactgtgt tggcgtacag                                                    20

<210>  7
<211>  21
<212>  DNA
<213>  Artificial Sequence

<220>
<223>  PLRG1 real-time PCT primer-F

<400>  7
cttaaagaga agggtcctca g                                                  21

<210>  8
<211>  21
<212>  DNA
<213>  Artificial Sequence

<220>
<223>  PLRG1 real-time PCT primer-R

<400>  8
ggtcctggtg ggtatggatg t                                                  21

<210>  9
<211>  21
<212>  DNA
<213>  Artificial Sequence

<220>
<223>  b-actin real-time PCT primer-F

<400>    9
gcaccacacc ttctacaatg a                                                21

<210>    10
<211>    21
<212>    DNA
<213>    Artificial Sequence

<220>
<223>    b-actin real-time PCT primer-R

<400>    10
tagcacagcc tggatagcaa c                                                21

<210>    11
<211>    21
<212>    RNA
<213>    Artificial Sequence

<220>
<223>    siPLRG1#1

<400>    11
uccggucaua augcuauuau u                                                21

<210>    12
<211>    21
<212>    RNA
<213>    Artificial Sequence

<220>
<223>    siPLRG1#2

<400>    12
agccaugaua cuacaauucg a                                                21

<210>    13
<211>    25
<212>    RNA
<213>    Artificial Sequence

<220>
<223>    siPLRG1#3

<400>    13
cgaggaggua cagaaacauu cugua                                            25

<210>    14
<211>    25
<212>    RNA
<213>    Artificial Sequence

<220>

<223>    siPLRG1#4

<400>    14
aaagagaagg guccucagaa ugcaa                                              25

<210>    15
<211>    25
<212>    RNA
<213>    Artificial Sequence

<220>
<223>    siPLRG1#5

<400>    15
cagaacuaua aagaucuggg acuug                                              25

<210>    16
<211>    25
<212>    RNA
<213>    Artificial Sequence

<220>
<223>    siPLRG1#6

<400>    16
uccagucagc uggaaaccag aaauu                                              25

<210>    17
<211>    25
<212>    RNA
<213>    Artificial Sequence

<220>
<223>    siPLRG1#7

<400>    17
cccaugacau guuuguagcu gauaa                                              25

<210>    18
<211>    25
<212>    RNA
<213>    Artificial Sequence

<220>
<223>    siPLRG1#8

<400>    18
ccaagaacuc ugcacugaug gcuaa                                              25

<210>    19
<211>    25
<212>    RNA
<213>    Artificial Sequence

<220>
<223>    siPLRG1#9

<400>    19
accguggaaa cucuacaggg uuauc                                          25

<210>    20
<211>    25
<212>    RNA
<213>    Artificial Sequence

<220>
<223>    siPLRG1#10

<400>    20
aagcugauaa aaccauuaaa guaua                                          25

<210>    21
<211>    21
<212>    DNA
<213>    Artificial Sequence

<220>
<223>    shPLRG1#1

<400>    21
ccatgatact acaattcgat t                                              21

<210>    22
<211>    21
<212>    DNA
<213>    Artificial Sequence

<220>
<223>    shPLRG1#2

<400>    22
gacttggcta gtggcaaatt a                                              21

<210>    23
<211>    73
<212>    DNA
<213>    Artificial Sequence

<220>
<223>    shPLRG1#1-F

<400>    23
cgaggtaccg gccatgatac tacaattcga ttctcgagaa tcgaattgta gtatcatggt    60

ttttagaatt ccg                                                       73

<210>    24
<211>    73
<212>    DNA
<213>    Artificial Sequence

<220>
<223>    shPLRG1#1-R


<400>    24
cggaattcta aaaaccatga tactacaatt cgattctcga gaatcgaatt gtagtatcat        60

ggccggtacc tcg                                                            73


<210>    25
<211>    73
<212>    DNA
<213>    Artificial Sequence

<220>
<223>    shPLRG1#2-F


<400>    25
cgaggtaccg ggacttggct agtggcaaat tactcgagta atttgccact agccaagtct        60

tttttgaatt ccg                                                            73


<210>    26
<211>    73
<212>    DNA
<213>    Artificial Sequence

<220>
<223>    shPLRG1#2-R


<400>    26
cggaattcaa aaaagacttg gctagtggca aattactcga gtaatttgcc actagccaag        60

tcccggtacc tcg                                                            73

## Description

### [Technical Field]

The present invention relates to a pharmaceutical composition for preventing or treating cancer containing pleiotropic regulator 1 (PLRG1) inhibitor as an active ingredient, a method for treating cancer including administering the composition to a subject, a composition for diagnosing cancer containing an agent for measuring the expression level of PLRG1, a method for providing information for diagnosing cancer including measuring the expression level of PLRG1, and a method for screening agents for preventing or treating cancer.

### [Background Art]

Cancer is characterized in that the growth of cancer cells is difficult to regulate compared to normal cells, and in that cancer cells can invade nearby tissues and sometimes become metastatic. Essentially, cancer is a disease associated with the regulation of tissue growth. When normal cells are transformed into cancer cells, there occurs a change in the expression of genes that regulate cell growth and division (Croce., The New England Journal of Medicine, 358(5): 502 to 511, 2008).

Examples of methods for cancer treatment may include surgical removal of tumor tissues; inhibition of expression of the genes essential for the survival of cancer, such as anti-apoptotic molecules, telomerase, growth factor receptor genes, signaling molecules, *etc.* including oncogenes, or induction of apoptosis by irradiation or administration of anticancer agents (Knudson AG, Nature reviews. Cancer 1(2): 157 to 162, 2001). However, the above methods also have a problem in that cancer cells as well as normal cells may be affected during the treatment, thereby causing side effects.

### [Disclosure]

### [Technical Problem]

The present inventors have made efforts to develop agents that can induce apoptosis in a cancer cell-specific manner without any side effects. As a result, they have confirmed that pleiotropic regulator 1 (hereinafter, PLRG1) is overexpressed in cancer cells rather than in normal cells, and inhibition of the overexpression can induce apoptosis in a cancer cell-specific manner, thereby completing the present invention.

### [Technical Solution]

An object of the present invention is to provide a pharmaceutical composition for preventing or treating cancer containing a PLRG1 inhibitor as an active ingredient.

Another object of the present invention is to provide a method for treating cancer including administering a pharmaceutical composition containing a PLRG1 inhibitor as an active ingredient to a subject.

Still another object of the present invention is to provide a composition for diagnosing cancer containing an agent for measuring the expression level of PLRG1.

Still another object of the present invention is to provide a method for providing information for diagnosing cancer, which includes (a) measuring the expression level of PLRG1 from an isolated biological sample; (b) comparing the expression level measured in step (a) with that of PLRG1 of a sample of a normal control group; and (c) when the expression level of PLRG1 from the isolated biological sample is greater than that of PLRG1 of a sample from the normal control group, establishing the presence of cancer.

Still another object of the present invention is to provide a method for screening agents for preventing or treating cancer, which includes (a) measuring the expression level of PLRG1 from a sample of an experimental animal having cancer, which is a control group; (b) administering a candidate material, which is expected to be able to treat cancer, to the experimental animal; (c) measuring the expression level of PLRG1 from a sample of the experimental animal to which the candidate material was administered, which is an experimental group; and (d) comparing the expression levels of PLRG1 measured in the experimental group and in the control group, and as a result, when the expression level measured in the experimental group is lower than that measured in the control group, establishing that the candidate material is able to be used as an agent for preventing cancer or an agent for treating cancer.

Still another object of the present invention is to provide a method for screening agents for preventing or treating cancer, which includes (a) treating isolated cancer cells capable of expressing PLRG1 with a candidate material for treating cancer; (b) measuring the expression level of PLRG1 in the cancer cells treated with the candidate material; and (c) when the expression level of PLRG1 measured in step (b) is lower than that of cancer cells not treated with the candidate material, establishing that the candidate material is able to be used as an agent for preventing cancer or an agent for treating cancer.

Still another object of the present invention is to provide a method for screening candidate materials to be used as an agent for preventing or treating cancer, which includes designing an anticancer candidate material inhibiting the transcription or translation of PLRG1 or inhibiting the activity of PLRG1 protein, based on the sequence of PLRG1 gene.

### [Advantageous Effects of the Invention]

PLRG1 is overexpressed in cancer cells and the inhibition of PLRG1 expression can induce cancer cell-specific apoptosis. Accordingly, the PLRG1 inhibitor of the present invention has an excellent effect as an anticancer agent without any side effects, and additionally, the PLRG1 inhibitor can be used for cancer diagnosis, screening of anticancer agents, *etc.* by measuring the expression levels of PLRG1.

### [Brief Description of Drawings]

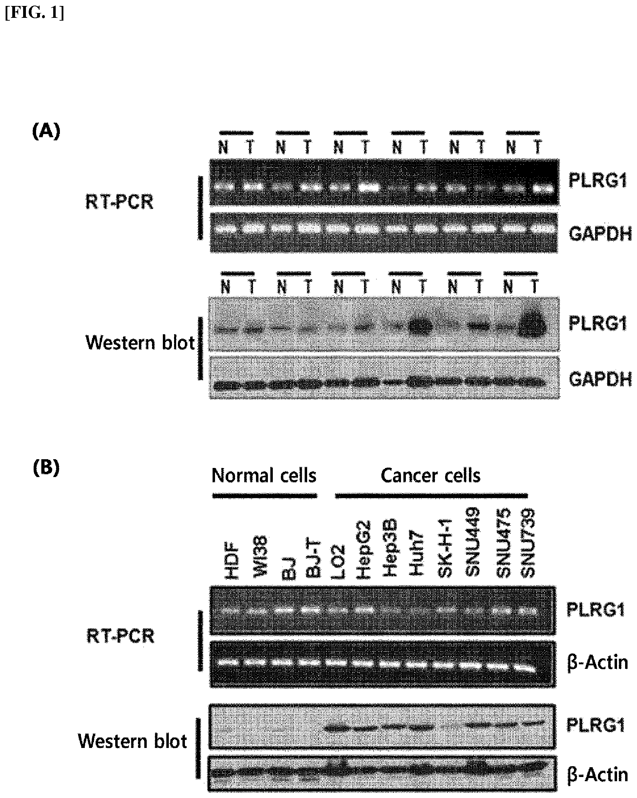

FIG. 1 confirms that the expression level of PLRG1 increases in liver cancer tissue and a liver cancer cell line. FIG. 1(A) shows the expression levels of PLRG1 in surrounding tissue of liver cancer (N) and liver cancer tissue (T) measured by RT-PCR and western blot; and FIG. 1(B) shows the expression levels of PLRG1 in a normal cell line and a cancer cell line measured by RT-PCR and western blot, in which LO2 represents immortalized cells.

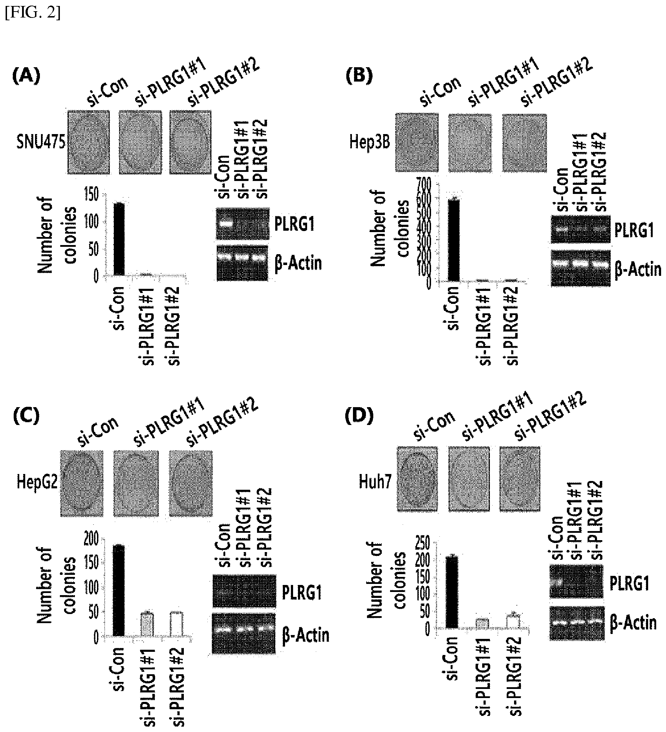

FIG. 2 confirms that clonal growth of cancer cells is inhibited by inhibiting the expression of PLRG1. The clonal growth was measured after transfection of Control-siRNA (si-Con) or PLRG1-siRNA (si-PLRG1#1, si-PLRG#2) into SNU475 (A), Hep3B (B), HepG2 (C), and Huh7 (D) cancer cell lines, respectively, and seeding the transfectants at a low-density. The colonies formed were stained with crystal violet (top) to quantify the number of colonies (bottom). The decreased expression level of PLRG1 and expression level of internal control β-actin were measured by RT-PCR.

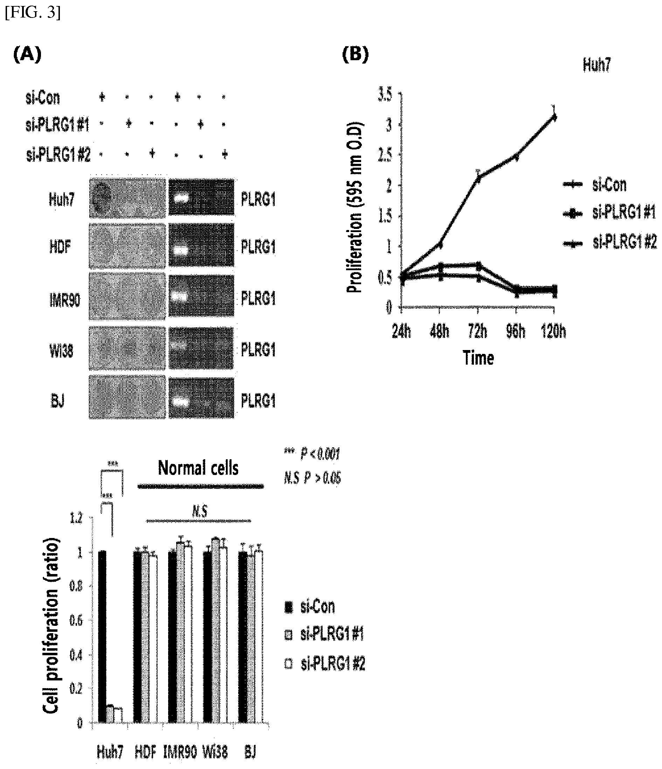

FIG. 3 confirms that cell growth is inhibited in a cancer cell-specific manner by inhibiting the expression of PLRG1. FIG. 3(A) shows the comparison results of cell growth, where Control-siRNA (si-Con) or PLRG1-siRNA (si-PLRG1#1, si-PLRG#2) was transfected into Huh7, HDF, IMR90, WI38, and BJ cells, respectively, and stained with crystal violet (top) after 5 days and the degree of coloration at OD 595 nm was quantified (bottom). The statistical significance of the difference in growth between cells treated with control-siRNA and those treated with PLRG1-siRNA was analyzed (Not significant, N.S. p>0.05; significant ***, p<0.001). FIG. 3(B) shows the results of cell growth curves which were prepared by measuring the effect of the reduced level of PLRG1 expression in Huh7 cells at 24-hour intervals. The expression levels of PLRG1 and β-actin were measured by RT-PCR.

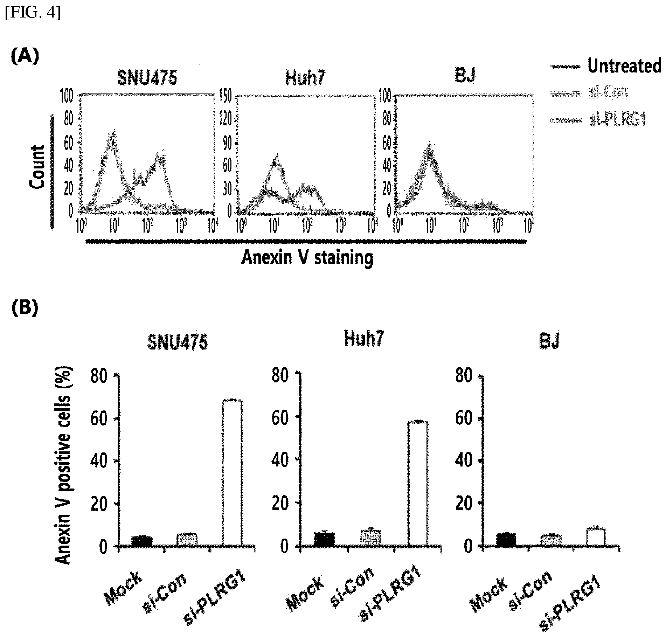

FIG. 4 confirms by annexin V staining that cancer-specific apoptosis is induced by inhibiting the expression of PLRG1. FIG. 4(A) shows the results where Control-siRNA (si-Con) or PLRG1-siRNA (si-PLRG#2) was transfected into Huh7, HDF, IMR90, WI38, and BJ cells, respectively, cultured for 96 hours, and dying cells were stained with annexin V. FIG. 4(B) shows results where annexin V-positive groups were quantified by measuring by FACS.

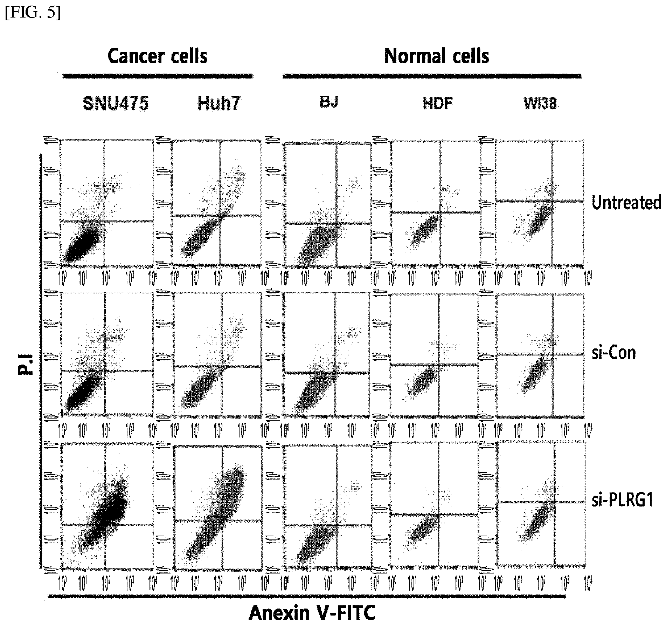

FIG. 5 confirms by the annexin V staining method that cancer-specific apoptosis is caused by inhibiting the expression of PLRG1. Control-siRNA (si-Con) or PLRG1-siRNA (si-PLRG#2) was transfected into normal cell lines (BJ, HDF, and WI38) and cancer cell lines (SNU475 and Huh7), respectively, and cultured for 96 hours. After staining with annexin V and PI, the PI-positive, annexin V-positive, and PI-annexin-double positive groups were measured by FACS.

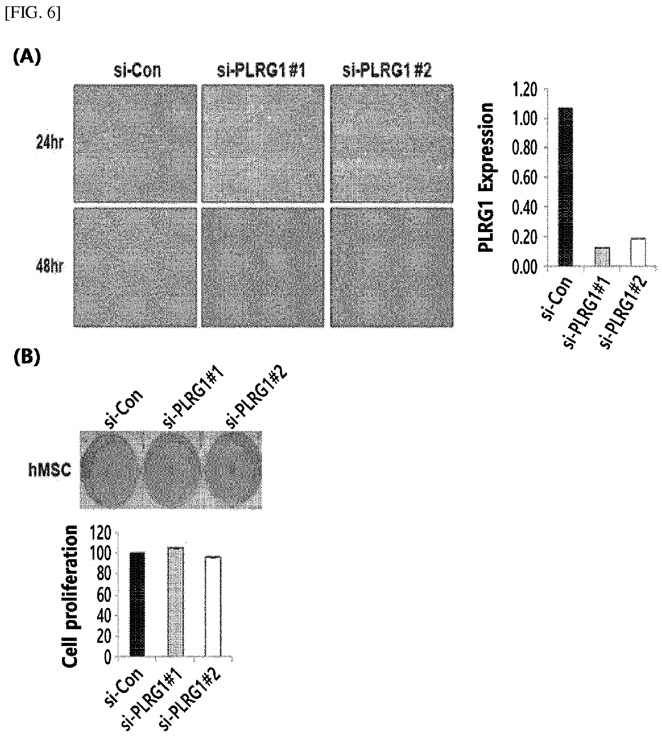

FIG. 6 confirms that the inhibition of PLRG1 expression does not have an effect on the growth of mesenchymal stem cells. FIG. 6(A) shows the images of cells where Control-siRNA (si-Con) or PLRG1-siRNA (si-PLRG1 #1 or si-PLRG #2) was transfected into mesenchymal stem cells and cultured for 7 days. FIG. 6(B) shows the results of cell growth rates which were measured by measuring the degree of color development at OD 595 nm after staining the cells with crystal violet (top). The cell growth rate was calculated based on the staining degree of cells treated with control-siRNA.

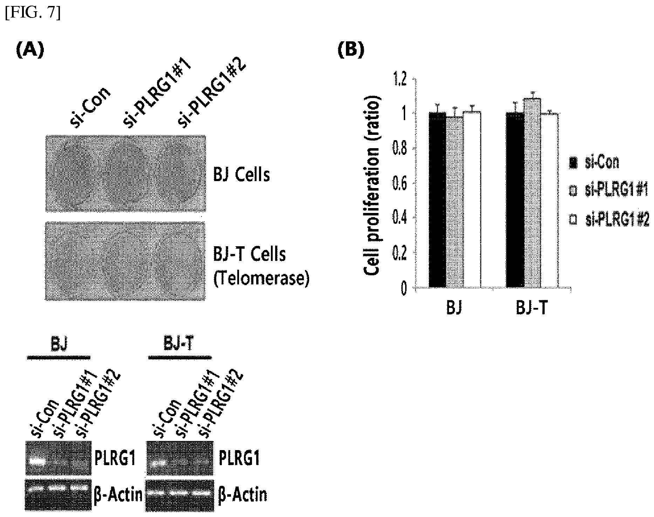

FIG. 7 confirms that the inhibition of PLRG1 expression does not have an effect on the growth of normal cells in which the expression of telomerase was induced. FIG. 7(A) shows the images of cells where Control-siRNA (si-Con) or PLRG1-siRNA (si-PLRG1 #1, si-PLRG #2) was transfected into BJ and BJ-T (telomerase-positive BJ) cells, cultured for 5 days, and stained with crystal violet. FIG. 7(B) shows the results of cell growth rates which were measured according to the degree of color development at OD 595 nm. The cell growth rate was calculated based on the staining degree of cells treated with control-siRNA. The expression levels of PLRG1 and β-actin were measured by RT-PCR.

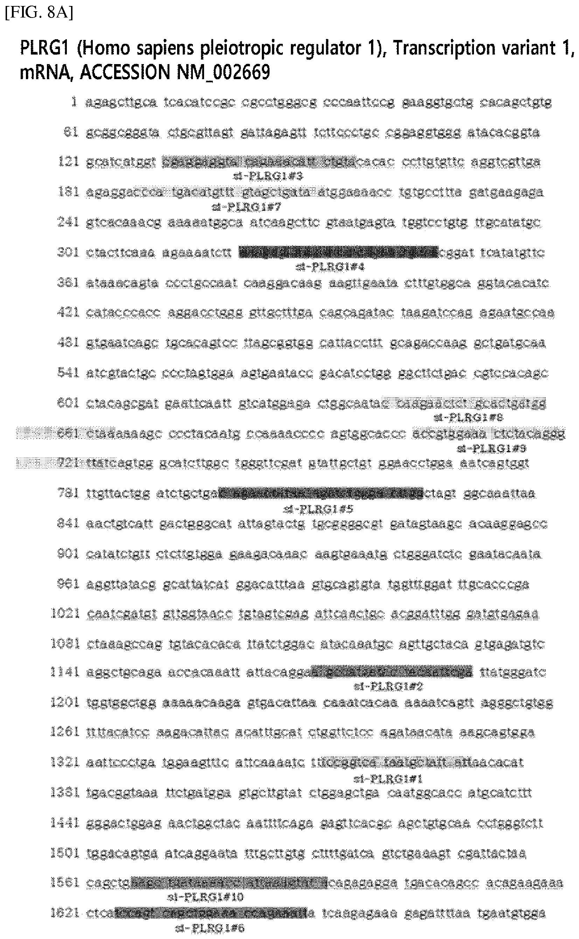

FIG. 8A shows the corresponding regions of 10 kinds of siRNAs prepared with reference to PLRG1.

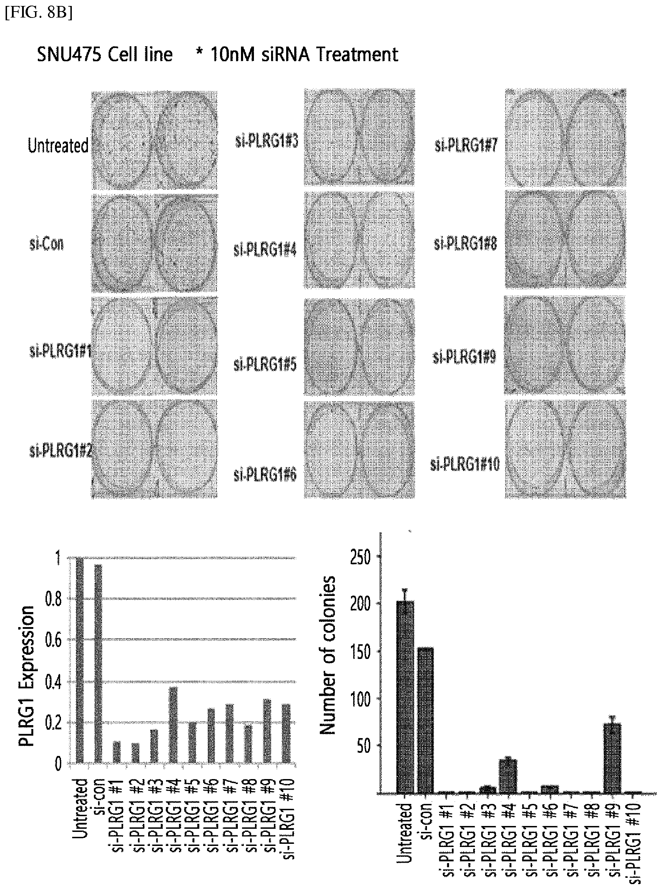

FIG. 8B confirms the expression levels of PLRG1 and the number of colonies when SNU475 cells were treated with Control-siRNA (si-Con) or PLRG1-siRNA (si-PLRG1 #1 to si-PLRG #10).

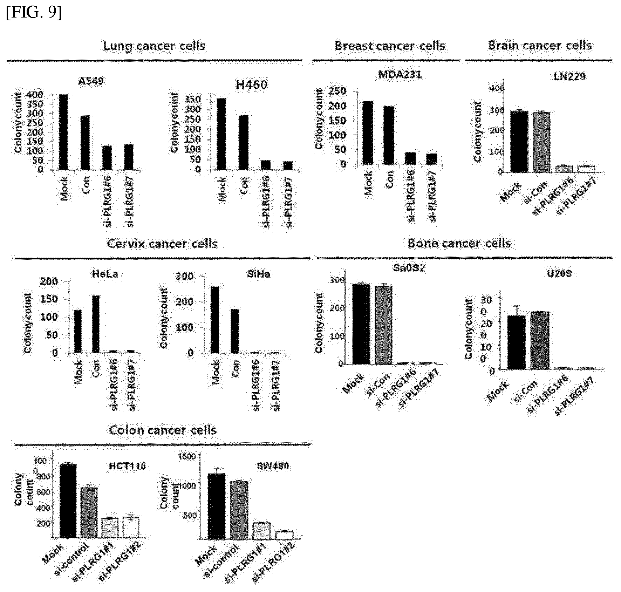

FIG. 9 confirms the number of colonies in various cancer cell lines according to the inhibition of PLRG1. The number of colonies was confirmed after transfecting Control-siRNA (si-Con) or PLRG1-siRNA to various cancer cells.

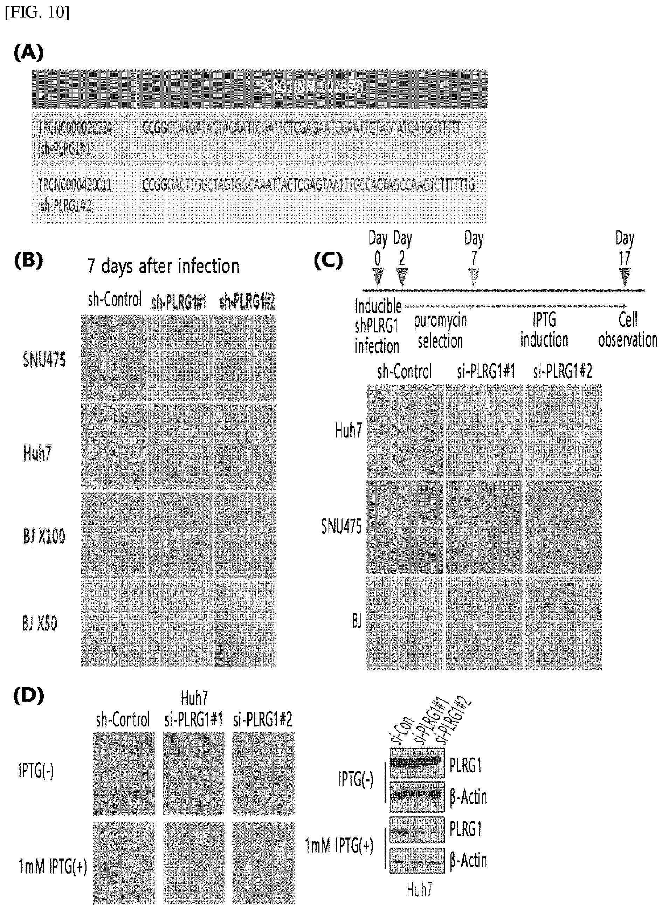

FIG. 10 confirms the system, where IPTG-inducible sh-PLRG1 is transfected into Huh7 and SNU475 cell lines. FIG. 10(A) shows the positions of target nucleotide sequences of human PLRG1 sh-RNA. FIG. 10(B) shows the images illustrating the death of cancer cells but not normal cells by infection of PLRG1 sh-RNA lentivirus, observed by microscope. FIG. 10(C) shows the images illustrating the infections by PLRG1 sh-RNA lentivirus and the cancer cell death by an inhibitory system of IPTG expression, observed by microscope. FIG. 10(D) shows the western blot results of protein expressions during cancer cell death.

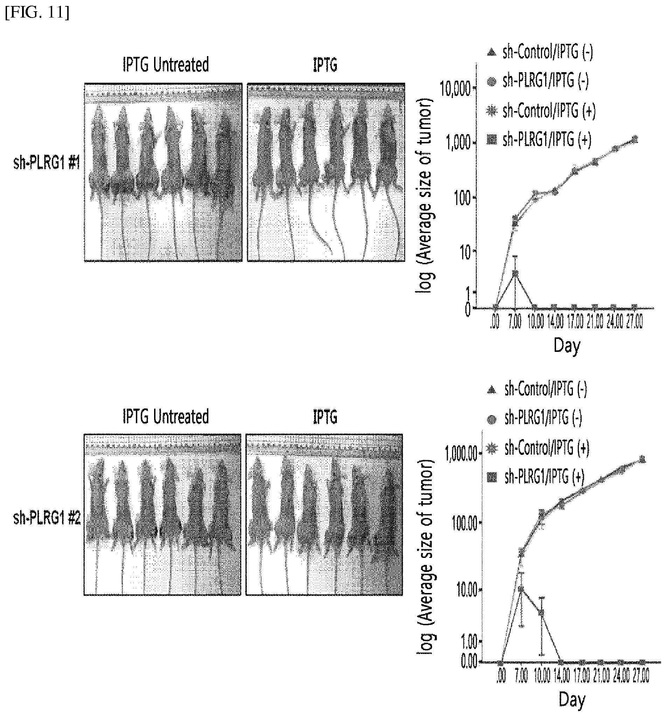

FIG. 11 confirms the effect of inhibiting PLRG1, in which the images on the left show the mice on the 27^{th} day after the injection of cells and the graphs on the right show the size of tumors in each mouse according to date.

### [Best Mode]

To achieve the above objects, in an aspect, the present invention provides a pharmaceutical composition for preventing or treating cancer containing a PLRG1 inhibitor as an active ingredient.

As used herein, the term "pleiotropic regulator 1 (PLRG1)" refers to a constitutive protein of the cell division cycle 5-like (CDC5L) complex, and PLRG1 has the role of pre-mRNA splicing as part of the spliceosome. PLRG1 plays a key role in determining the site for alternative splicing. There are several reports regarding the developmental stage, but no correlation between PLRG1 and cancer has been reported.

In a specific embodiment of the present invention, the present inventors have confirmed for the first time that the levels of PLRG1 expression in tumor tissues and cancer cell lines are higher than those in normal tissues and normal cell lines (FIG. 1) and have thereby confirmed that PLRG1 is suitable as a target for cancer treatment.

As used herein, the term "PLRG1 inhibitor" collectively refers to all of the agents that can decrease the expression or activity of PLRG1, and specifically, a PLRG1 inhibitor may include all of the agents that can decrease the expression level or activity of PLRG1, by reducing the PLRG1 expression at the transcription level or preventing activity of PLRG1, by directly acting on PLRG1 or indirectly acting on ligands thereof, *etc.*

Examples of the inhibitors of PLRG1 may include compounds, nucleic acids, peptides, viruses, vectors containing the nucleic acids, *etc.* that can inhibit the expression or activity of PLRG1 by targeting PLRG1, and they can be used without limitation regardless of their forms. Specifically, the inhibitors of PLRG1 may be oligonucleotides that inhibit the expression of PLRG1 mRNA, antibodies or antigen-binding fragments thereof that inhibit the activity of PLRG1 proteins, more specifically, the oligonucleotides that inhibit the expression of PLRG1 mRNA may be antisense oligonucleotides, aptamers, shRNAs, or siRNAs which are specific to the PLRG1 mRNA, and even more specifically, the siRNAs may be double-stranded siRNAs which have any one oligonucleotide among SEQ ID NOS: 11 to 20 and a complementary oligonucleotide thereof, and the siRNAs may be those which have the oligonucleotide sequence of SEQ ID NO: 21 or 22, but the PLRG1 inhibitors are not limited thereto.

As used herein, the term "antisense oligonucleotide" refers to DNA, RNA, or a derivative thereof containing a complementary sequence to a particular mRNA sequence, and which binds to a complementary sequence in the mRNA and acts to inhibit the translation of the mRNA into a protein. An antisense oligonucleotide sequence refers to a DNA or RNA sequence which is complementary to the PLRG1 mRNA and is able to bind to the mRNA. An antisense oligonucleotide can inhibit essential activities with respect to the translation, translocation into the cytoplasm, and maturation of the PLRG1 mRNA, or all other biological functions. The length of an antisense oligonucleotide may be 6 to 100 nucleotides, preferably 8 to 60 nucleotides, and more preferably 10 to 40 nucleotides. The antisense oligonucleotide may be synthesized *in vitro* by a conventional method and administered into the living body or may be synthesized *in vivo.*

An example of synthesizing an antisense oligonucleotide *in vitro* is to use RNA polymerase I. An example of synthesizing an antisense RNA *in vivo* is to use a vector in which the origin of the multiple cloning site (MCS) is in the opposite direction so that the antisense RNA can be transcribed. It is preferred that a translation stop codon be present in the antisense RNA sequence so that the antisense RNA cannot be translated into a peptide sequence. The design of the antisense oligonucleotide that can be used in the present invention can easily be produced by a method known in the art with reference to the nucleotide sequence of PLRG1.

As used herein, the term "aptamer" refers to a nucleic acid molecule which is a single-stranded oligonucleotide having a size of 20 to 60 nucleotides and has a binding activity to a particular target molecule. An aptamer can have various three-dimensional structures depending on its sequence, and can have high affinity for a specific material as in an antigen-antibody reaction. The aptamer can inhibit the activity of a particular target molecule by binding to the particular target molecule. The aptamer of the present invention may be RNA, DNA, a modified nucleic acid, or a mixture thereof, and additionally, the aptamer of the present invention may be in a linear or circular form. Preferably, the aptamer may have the role of inhibiting the activity of PLRG1 by binding to PLRG1. The aptamer can be prepared from a sequence of PLRG1 by a method known to a skilled person in the art.

As used herein, the terms "siRNA" and "shRNA" refer to nucleic acid molecules capable of mediating RNA interference or gene silencing. Since siRNA and shRNA can inhibit the expression of a target gene, they can be used as a method for efficient gene knockdown or gene therapy. The shRNA is a hairpin structure formed by the binding between complementary sequences within a single-stranded oligonucleotide. The shRNA is cleaved *in vivo* by a dicer and becomes an siRNA, which is a double-stranded oligonucleotide having a small RNA fragment of 21 to 25 nucleotides in size, and it can specifically bind to mRNA having a complementary sequence and thereby inhibit the expression of the mRNA.

Therefore, whether to use an shRNA or siRNA can be determined by a skilled person in the art, and a similar effect of reducing expression can be expected if the mRNA sequences to which they are targeted are the same. For the purpose of the present invention, shRNA or siRNA may be allowed to specifically act on PLRG1 and induce RNA interference (RNAi) by cleaving PLRG1 mRNA molecules, and thereby PLRG1 expression can be inhibited. The siRNA may be chemically or enzymatically synthesized. The method for preparing the siRNA is not particularly limited, but any method known in the art can be used. Examples of these methods may include direct synthesis of siRNA, synthesis of siRNA by *in vitro* transcription, cleavage of long double-stranded RNA synthesized by *in vitro* transcription using an enzyme, expression by intracellular delivery of an shRNA expression plasmid or viral vector, expression by intracellular delivery of a polymerase chain reaction (PCR)-induced siRNA expression cassette, *etc.,* but the methods are not limited thereto.

In particular, the siRNA with respect to the PLRG1 of the present invention may be a double-stranded molecule consisting of any one oligonucleotide of SEQ ID NOS: 11 to 20 and an oligonucleotide complementary thereof, but the siRNA is not limited thereto. Additionally, the shRNA may be one which has an oligonucleotide sequence of SEQ ID NO: 21 or SEQ ID NO: 22, but the shRNA is not limited thereto.

As used herein, the term "antibody" refers to a material that reacts to an antigen (*i.e*., an external material) that has invaded an *in vivo* immune system by the circulation of blood or lymph, and it is a globulin-based protein formed in lymphoid tissue and is also called immunoglobulin. Antibodies are proteins that are produced by B cells and flowed into body fluids. Antibodies specifically bind to antigens, and each antibody molecule consists of two heavy chains and two light chains, in which each heavy chain and each light chain has a variable region at its N-terminal end. Each variable region consists of three complementarity determining regions (CDRs) and four framework regions (FRs), in which the complementarity determining regions determine the antigen-binding specificity of an antibody and are present as relatively short peptide sequences maintained at the structure-forming sites of the variable region. For the purpose of the present invention, the antibody may be an antibody that can bind to PLRG1 and inhibit the activity of PLRG1.

As used herein, the term "cancer" refers to a disease associated with the regulation of cell death which is caused by excessive cell proliferation when the normal apoptotic balance is broken. These abnormal excessively proliferated cells sometimes invade adjacent tissues and organs and form masses and destroy or deform the normal structures in the body, and this state is called cancer. Generally, a tumor refers to a mass that has grown abnormally due to autonomous overgrowth of body tissue, and can be divided into benign tumors and malignant tumors. Malignant tumors grow much faster than benign tumors, and metastasis occurs as they infiltrate the surrounding tissues, thereby threatening life. These malignant tumors are commonly referred to as "cancer". The types of cancer may include cerebrospinal fluid tumor, brain tumor, head and neck cancer, lung cancer, breast cancer, thymoma, mesothelioma, esophageal cancer, pancreatic cancer, colon cancer, liver cancer, stomach cancer, kidney cancer, biliary tract cancer, kidney cancer, bladder cancer, prostate cancer, testicular cancer, germ cell tumor, ovarian cancer, uterine cervix cancer, endometrial cancer, colorectal cancer, lymphoma, acute leukemia, chronic leukemia, multiple myeloma, sarcoma, malignant melanoma, and skin cancer, but the types of cancer of the present invention are not limited thereto. Additionally, in the present invention, the cancer may be those which are overexpressed compared to the normal tissues, but the cancer is not limited thereto.

The prevention or treatment of cancer may be achieved by cancer cell-specific apoptosis. The apoptosis is a type of cell death that can be distinguished from necrosis in which cells are physically destroyed, and it is also called programmed cell death and refers to a process by which DNA becomes damaged or unnecessary cells die by themselves.

In a specific embodiment of the present invention, when the PLRG1 expression was reduced by treating siRNA for PLRG1 on cells, it was confirmed that monoclonal growth was reduced and cell proliferation was inhibited in the cancer cell lines, whereas the cell proliferation was not affected in the normal cell line (FIG. 3). Additionally, it was confirmed by staining the cells treated with the siRNA for PLRG1 with annexin V alone or annexin V and PI that the above results were due to cancer cell-specific apoptosis (FIGS. 4 and 5). Furthermore, it was confirmed that these cancer cell-specific effects due to the reduction of PLRG1 did not occur even in normal cells transfected with mesenchymal stem cells and telomerase activity, thus confirming that the inhibition of PLRG1 can induce cancer cell-specific apoptosis in a very strict manner (FIGS. 6 and 7). Furthermore, the above results were shown to occur in the same manner *in vivo* using the IPTG-inducible-sh-PLRG1 system in a mouse animal model (FIG. 11). This suggests that when a PLRG1 inhibitor is used as an anticancer agent, it can induce cancer cell-specific apoptosis without affecting normal cells and without side effects.

As used herein, the term "treatment" refers to clinically intervening to alter the natural course for an individual or cells to be treated, and this can be performed while the conditions of clinical pathology progress or for the prevention of the conditions of the same. The desired therapeutic effects include prevention of occurrence or recurrence of diseases, alleviation of symptoms, deterioration of all of the direct/indirect pathological results associated with the diseases, prevention of metastasis, reduction of progress of diseases, alleviation or temporary relief of disease conditions, and amelioration of diseases or prognosis. Preferably, in the present invention, the treatment includes all of the actions that improve cancer progress by administering a composition containing a material inhibiting PLRG1. Additionally, the term "prevention" refers to all of the actions that inhibit or delay the occurrence of cancer by administering a composition containing a material inhibiting PLRG1 according to the present invention.

The pharmaceutical composition of the present invention may further include an appropriate carrier, excipient, or diluent which are conventionally used in the preparation of pharmaceutical compositions. The composition containing a pharmaceutically acceptable carrier may be prepared in various formulations for oral or parenteral administration. The formulations may be prepared using a commonly used excipient such as a filler, an extender, a binder, a humectant, a disintegrant, a surfactant, a diluent *etc.* Solid formulations for oral administration may include tablets, pills, powders, granules, capsules, *etc.,* and these solid formulations may be prepared by adding at least one excipient (*e.g*., starch, calcium carbonate, sucrose or lactose, gelatin, *etc*.). Additionally, a lubricant, such as magnesium stearate, talc, *etc.,* may be used in addition to the simple excipient. Liquid formulations for oral administration may include suspensions, liquid medicines for internal use, emulsions, syrups, *etc.,* and various excipients such as humectants, sweeteners, fragrances, preservatives, *etc.,* may be used in addition to the simple diluents such as water and liquid paraffin. Formulations for parenteral administration may include sterile aqueous solutions, non-aqueous solvents, suspensions, emulsions, lyophilized formulations, and suppositories. Examples of the non-aqueous solvents and suspensions may include vegetable oils such as propylene glycol, polyethylene glycol, and olive oil, an injectable ester such as ethyl oleate, *etc.* Examples of the bases for suppositories may include Witepsol, macrogol, Tween 61, cacao butter, laurinum, glycerogelatin, *etc.*

Additionally, the pharmaceutical composition of the present invention may have any one formulation selected from the group consisting of tablets, pills, powders, granules, capsules, suspensions, liquid medicines for internal use, emulsions, syrups, sterile aqueous solutions, non-aqueous solvents, suspensions, emulsions, lyophilized formulations, and suppositories, but the formulation is not limited thereto.

Another aspect of the present invention provides a method for treating cancer including administering a pharmaceutical composition containing a PLRG1 inhibitor as an active ingredient to a subject.

As used herein, the term "subject" refers to all of the animals including humans which have a cancer disease or in which a cancer disease has occurred. Cancer including liver cancer can be alleviated or treated by administering a pharmaceutical composition of the present invention to a subject. The alleviation refers to all of the actions which ameliorate or beneficially change the cancer disease by administering the pharmaceutical composition of the present invention.

The pharmaceutical composition of the present invention is administered in a pharmaceutically effective amount.

As used herein, the term "administration" refers to introduction of the pharmaceutical composition of the present invention to a patient by an appropriate manner, and the composition may be administered by various oral or parenteral routes as long as the composition can arrive at a target tissue.

The pharmaceutical composition may be appropriately administered to a subject according to the conventional administration method, route, and amount used in the art according to the purposes and uses thereof. The pharmaceutical composition may be administered orally, parenterally, subcutaneously, intraperitoneally, and intranasally. Examples of the parenteral administration include intramuscularly, intravenously, intraperitoneally, and intradermally. Additionally, appropriate administration frequency and dose may be selected according to methods known in the art. In fact, the administration frequency and dose of the pharmaceutical composition of the present invention may be appropriately determined by various factors, such as the types of symptoms to be treated, administration routes, sex, health conditions, diets, age, and body weight of a subject, and severity of a disease.

As used herein, the term "pharmaceutically effective amount" refers to an amount sufficient for the inhibition or alleviation of the increase in vascular permeability at a reasonable benefit/risk ratio applicable to a medical use, and the level of the effective dose may be determined based on the factors including the kind of a subject, severity of illness, age, sex, drug activity, drug sensitivity, duration of administration, administration route and dissolution rate, length of treatment, factors including drug(s) to be used simultaneously in combination, and other factors well known in the medical field. The composition of the present invention may be administered as an individual therapeutic agent, in combination with other therapeutic agents, or sequentially or simultaneously with a conventional therapeutic agent(s), and may be administered once or multiple times. It is important to administer an amount to obtain the maximum effect with a minimum amount without adverse effects considering all of the factors described above, and these factors can easily be determined by those skilled in the art.

Still another aspect of the present invention provides a composition for diagnosing cancer containing an agent for measuring the expression level of PLRG1.

Still another aspect of the present invention provides a method for providing information for diagnosing cancer, which includes (a) measuring the expression level of PLRG1 from an isolated biological sample; (b) comparing the expression level measured in step (a) with that of PLRG1 of a sample of a normal control group; and (c) when the expression level of PLRG1 from the isolated biological sample is greater than that of PLRG1 of a sample from the normal control group, establishing the presence of cancer. By detecting the cancer marker PLRG1 through the present invention, information necessary for cancer diagnosis can be provided, and thereby cancer can be diagnosed.

As used herein, the term "diagnosis" refers to identifying the presence or characteristics of a cancer disease by measuring the presence or absence of the PLRG1 of the invention in a biological sample or tissue sample. Additionally, the term "marker or diagnostic marker" refers to a material that can diagnose a subject having cancer cells or a cancer disease by distinguishing from normal cells or a normal subject, and it includes organic biomolecules such as polypeptides, proteins or nucleic acids (*e.g*., mRNA, *etc*.), lipids, glycolipids, glycoproteins or saccharides (*e.g*., monosaccharides, disaccharides, oligosaccharides, *etc*.), which show an increase or decrease in cells or a subject having cancer compared to normal cells. For the purpose of the present invention, the cancer diagnostic marker of the present invention is PLRG1, which shows a particularly high expression level in cancer cells compared to normal cells or normal tissue cells.

In the present invention, the measurement of PLRG1 expression level may be to measure the expression level of PLRG1 mRNA or the expression level of PLRG1 protein.

As used herein, the term "measurement of expression level of mRNA" refers to a process of confirming the presence of mRNA of a cancer marker gene in a biological sample and an expression level thereof for the diagnosis of cancer, and the expression level can be confirmed by measuring the amount of mRNA. Examples of the methods for this analysis include RT-PCR, competitive RT-PCR, real-time RT-PCR, RNase protection assay (RPA), northern blotting, DNA chips, *etc.,* but the methods are not limited thereto.

As used herein, "measurement of expression level of protein" refers to a process of confirming the presence of a protein expressed from a cancer marker gene in a biological sample and an expression level thereof for the diagnosis of cancer, and the amount of the protein can be confirmed using an antibody that specifically binds to the protein of the gene. Examples of the methods for this analysis include western blot, enzyme linked immunosorbent assay (ELISA), radioimmunoassay (RIA), radioimmunodiffusion, Ouchterlony immunodiffusion, rocket immunoelectrophoresis, immunohistochemistry, immunoprecipitation assay, complement fixation assay, FACS, protein chips, *etc.,* but the methods are not limited thereto.

In an embodiment, the agent for measuring the mRNA level may be a pair of primers, a probe, or an anti-sense nucleotide with respect to the PLRG1 mRNA, and those skilled in the art can easily design the primers, probe, or antisense nucleotide sequence based on the polynucleotide sequence of PLRG1 of the present invention. In another embodiment, the agent for measuring the protein level may be an antibody.

In the present invention, the potential of PLRG1 as a cancer diagnostic marker was confirmed, and as a result, it was confirmed that the measurement of the PLRG1 levels enables the diagnosis of cancer including liver cancer.

Still another aspect of the present invention provides a method for screening agents for preventing or treating cancer, which includes (a) measuring the expression level of PLRG1 from a sample of an experimental animal having cancer, which is a control group; (b) administering a candidate material, which is expected to be able to treat cancer, to the experimental animal; (c) measuring the expression level of PLRG1 from a sample of the experimental animal to which the candidate material was administered, which is an experimental group; and (d) comparing the expression levels of PLRG1 measured in the experimental group and in the control group, and as a result, when the expression level measured in the experimental group is lower than that measured in the control group, establishing that the candidate material is able to be used as an agent for preventing cancer or an agent for treating cancer.

Still another aspect of the present invention provides a method for screening agents for preventing or treating cancer, which includes (a) treating isolated cancer cells capable of expressing PLRG1 with a candidate material for treating cancer; (b) measuring the expression level of PLRG1 in the cancer cells treated with the candidate material; and (c) when the expression level of PLRG1 measured in step (b) is lower than that of cancer cells not treated with the candidate material, establishing that the candidate material is able to be used as an agent for preventing cancer or an agent for treating cancer.

The expression level of the gene of the present invention or the expression level of the protein encoded by the gene in a cell in the absence of a candidate material capable of preventing or treating cancer is measured, and additionally, the expression level of the gene of the present invention or the expression level of the protein encoded by the gene in the presence of the candidate material is measured and the two expression levels compared, and as a result, if the expression level of the gene of the present invention or the expression level of the protein encoded by the gene is reduced in the presence of the candidate material compared to the expression level of the gene of the present invention or the expression level of the protein encoded by the gene in the absence of the candidate material, the candidate material is expected to be used as an agent for preventing or treating cancer.

The screening method may be performed *in vivo* or *in vitro,* but the method is not particularly limited thereto. The candidate material may be a known material or novel material, and for example, a large-scale screening may be performed using plant extracts or chemical libraries. Through the above process, agents for preventing or treating cancer by inducing cancer cell-specific apoptosis via inhibition of the expression or activity of PLRG1 can be discovered.

Still another aspect of the present invention provides a method for screening candidate materials to be used as an agent for preventing or treating cancer, which includes designing an anticancer candidate material inhibiting the transcription or translation of PLRG1 or inhibiting the activity of PLRG1 protein, based on the sequence of PLRG1 gene.

As described above, in the present invention, it was confirmed in various aspects both *in vivo* and *in vitro* that the inhibition of PLRG1 expression results in inhibiting proliferation of cancer cells in a cancer cell-specific manner followed by apoptosis. That is, it was first confirmed in the present invention that the inhibition of expression of PLRG1 is associated with a cancer cell-specific anticancer effect. Accordingly, those skilled in the art can recognize through the present invention that a material inhibiting the transcription, translation, or activity of the PLRG1 protein may have anticancer activity. As such, it should be obvious that the siRNA and shRNA with regard to PLRG1, whose effects to PLRG1 were confirmed in Examples of the present invention, can be regarded as embodiments simply confirming the technical concept, and those skilled in the art can design candidate materials of agents for preventing or treating cancer capable of inhibiting the expression, activity, *etc.* of PLRG1, based on the gene sequence information, through various methods known in the art. In a specific embodiment thereof, an siRNA or shRNA inhibiting PLRG1 can be prepared based on the gene sequence of PLRG1, or compounds capable of inhibiting PLRG1 can be predicted based on the structure of the protein translated from the PLRG1 gene through a computer program, *etc.* The candidate materials selected by the screening method may be used for selecting effective materials through *in vivo* and *in vitro* screening.

### [DETAILED DESCRIPTION OF THE INVENTION]

Hereinafter, the present invention will be described in more detail with reference to the following Examples. However, these Examples are for illustrative purposes only and the invention is not limited by these Examples. The Examples of the present invention are provided to enable those skilled in the art to more fully understand the present invention.

### Example 1. Primers and siRNA sequence

RT-PCR and real-time PCR primers used for gene expression are as follows.

### [RT-PCR]

PLRG1 forward primer: ACCGTCCAC AGCCTACAGCGA (SEQ ID NO: 1)
PLRG1 reverse primer: ACTATCACGCCCCGCACAGT (SEQ ID NO: 2)
GAPDH forward primer: GTCAGTGGTGGACCTGACCT (SEQ ID NO: 3)
GAPDH reverse primer: TGCTGTAGCCAAATTCGTTG (SEQ ID NO: 4)
β-actin forward primer: GGACTTCGAGCAAGAGATGG (SEQ ID NO: 5)
β-actin reverse primer: AGCACTGTGTTGGCGTACAG (SEQ ID NO: 6)

### [Real-time PCR]

PLRG1 forward primer: CTTAAAGAGAAGGGTCCTCAG (SEQ ID NO: 7)
PLRG1 reverse primer: GGTCCTGGTGGGTATGGATGT (SEQ ID NO: 8)
β-actin forward primer: GCACCACACCTTCTACAATGA (SEQ ID NO: 9)
β-actin reverse primer: TAGCACAGCCTGGATAGCAAC (SEQ ID NO: 10)

The siRNA and shRNA used to inhibit PLRG1 expression are as follows.
siPLRG1 #1: uccggucauaaugcuauuauu (SEQ ID NO: 11)
siPLRG1 #2: agccaugauacuacaauucga (SEQ ID NO: 12)
siPLRG1 #3: cgaggagguacagaaacauucugua (SEQ ID NO: 13)
siPLRG1 #4: aaagagaaggguccucagaaugcaa (SEQ ID NO: 14)
siPLRG1 #5: cagaacuauaaagaucugggacuug (SEQ ID NO: 15)
siPLRG1 #6: uccagucagcuggaaaccagaaauu (SEQ ID NO: 16)
siPLRG1 #7: cccaugacauguuuguagcugauaa (SEQ ID NO: 17)
siPLRG1 #8: ccaagaacucugcacugauggcuaa (SEQ ID NO: 18)
siPLRG1 #9: accguggaaacucuacaggguuauc (SEQ ID NO: 19)
siPLRG1 #10: aagcugauaaaaccauuaaaguaua (SEQ ID NO: 20)
shPLRG1 #1: CCATGATACTACAATTCGATT (SEQ ID NO: 21)
shPLRG1 #2: GACTTGGCTAGTGGCAAATTA (SEQ ID NO: 22)

### Example 2. Cultivation of cancer cells, normal cells, and mesenchymal stem cells

Huh7 and SNU475 cells, which are human liver cancer cell lines purchased from ATCC, were cultured in RPMI medium containing 10% fetal bovine serum (FBS). Hep3B and HepG2 cells, which are different human liver cancer cell lines, were cultured in MEM medium containing 10% FBS. HDF, IMR90, WI38, BJ, and BJ-T cells, which are normal cells, were cultured in DMEM medium. The human mesenchymal stem cells purchased from Lonza Inc. were cultured in human mesenchymal stem cell growth BulletKit™ medium (MSCGM).

### Example 3. RNA isolation and RT-PCR

RNA was extracted using the Qiagen RNeasy Mini Kit and RNA concentrations were measured using the ND-1000 spectrophotometer (NanoDrop Technologies, Inc. Wilmington, DE, USA). Additionally, complementary DNA (cDNA) was synthesized using the iScript™ cDNA Synthesis Kit and reverse transcriptase-polymerase chain reaction (RT-PCR) was performed using the Maxime PCR PreMix kit (Intron Biotechnology).

### Example 4. Protein isolation and western blot

Proteins were extracted from the tissues of a liver cancer patient and the adjacent tissues thereof using a lysis buffer, and protein expression levels were measured using PLRG1 antibodies (SIGMA) and GAPDH antibodies (Santacruz). Western blot was performed for each cell line in the same manner as described above. As the control group, β-actin antibodies (Santacruz) were used.

### Example 5. Measurement of inhibition of monoclonal growth using PLRG1 inhibitor

The present inventors used an siRNA, which is a representative for PLRG1, as an inhibitor for the inhibition of PLRG1 expression. The siPLRG1 #1 to siPLRG #10 used were purchased from Qiagen and control-siRNA was purchased from Mbiotech. Each cell was inoculated into a 60 mm culture dish and then control-siRNA and two types of PLRG1-siRNA were transfected thereto, respectively. After 24 hours, 1,000 cells were counted and re-inoculated thereto, and after 10 days, the cells were stained with crystal violet. The stained colonies were calculated to quantify monoclonal growth.

### Example 6. Inhibition of cell growth using PLRG1 inhibitor

Each cell was inoculated into a 24-well culture dish and then control-siRNA and two types of PLRG1-siRNA were transfected thereto, respectively. After 5 days, the cells were stained with crystal violet, dissolved in glacial acetic acid, and the absorbance was measured at OD 595 nm. Each experiment was repeated 3 times. The cell growth of Huh7 cell line was measured at 24, 48, 72, 96, and 120 hours.

### Example 7. Induction of apoptosis using PLRG1 inhibitor

Each cell was inoculated into a 60 mm culture dish and then control-siRNA and PLRG1-siRNA were treated thereto, respectively. After 72 hours, the cells were all collected and stained for 15 minutes after adding annexin V-FITC thereto, and the degree of apoptosis was measured using a flow cytometer.

For a double-staining, the cells were stained for 15 minutes after simultaneously adding annexin V-FITC and propidium iodide (PI) thereto, and the degree of apoptosis was measured using a flow cytometer.

### Experimental Example 1. Confirmation of expression level of PLRG1 in liver cancer tissue and liver cancer cell line

The amount of PLRG1 mRNA present in each of adjacent non-neoplastic liver tissues and liver cancer tissues was measured by an RT-PCR method. As a result, it was confirmed that the PLRG1 mRNA was overexpressed in the liver tissues compared to that in the adjacent non-neoplastic liver tissues (FIG. 1). As a result of western blot analysis of the amount of PLRG1 protein, it was also confirmed that the amount of PLRG1 protein was significantly increased in the liver cancer tissues (FIG. 1A).

In the experiment using cell lines, it was also confirmed that the expression level of PLRG1 in the cancer cell line was greater compared to that in the normal cell line (FIG. 1B).

### Experimental Example 2. Confirmation of inhibition of monoclonal growth by inhibition of PLRG1 expression in cancer cell line

From the results of Experimental Example 1, it was predicted that PLRG1 could play a role with regard to cancer progression such as cancer growth, *etc.,* and to confirm this, the effect of inhibition of PLRG1 expression on the monoclonal growth of cancer cells was measured.

As a result, it was confirmed that when the PLRG1 expression was reduced by treating the cancer cell line SNU475 with PLRG1-siRNA, the colony formation was significantly inhibited compared to the control group treated with the control-siRNA (FIG. 2A). The inhibition of colony formation in cancer cells by inhibition of PLRG1 expression as such was also observed in Hep3B, HepG2, and Huh7 cancer cell lines (FIGS. 2B, 2C, and 2D). Additionally, the inhibition of monoclonal growth by the reduction of PLRG1 expression was similarly observed in other experiments where two different PLRG1-siRNAs (#1 and #2) were used. From these results, it was confirmed that the reduction in PLRG1 expression can inhibit monoclonal growth in various kinds of cancer cells.

### Experimental Example 3. Confirmation of inhibitory effect against PLRG1 expression in normal cell line

To examine whether an inhibitor of PLRG1 expression can affect the growth of a normal cell line, the growth of a cancer cell line and a normal cell line was measured after treating each cell line with PLRG1-siRNA and the results were compared. As a result of the measurement of the cell growth by a crystal violet staining method, it was confirmed that cell growth was not achieved by the inhibition of PLRG1 expression (FIG. 3A), as shown in the experiment of colony formation, but rather the cell growth was reduced (FIG. 3B). However, in the cases of HDF, IMR90, WI38, and BJ normal cell lines which were applied to the experiment, the phenomenon of the reduction in cell growth by the inhibition of PLRG1 expression compared to the control group treated with control-siRNA was not observed (FIG. 3A, p>0.05). From these results, it was confirmed that the inhibition of cell growth by the reduction of PLRG1 expression was cancer cell-specific.

### Experimental Example 4. Confirmation of apoptosis by inhibition of PLRG1 expression

To examine whether the inhibition of cell growth by the inhibition of PLRG1 expression is associated with cell death, the cells stained with annexin-V (*i.e*., an apoptosis marker) were measured. In the cases of Huh7 and SNU475 cancer cell lines, the intensity of annexin V staining was significantly increased when the cells were treated with PLRG1-siRNA compared to the cell line treated with control-siRNA, whereas in the case of the BJ cell (*i.e*., a normal cell line), there was no difference compared to the control group (FIGS. 4A and 4B).

For more accurate analysis, the change in cells double-stained with annexin V and PI was analyzed using FACS. As a result, it was confirmed that the number of cells simultaneously stained with annexin V and PI (used as late apoptosis markers) was significantly increased in the cancer cell lines (SNU475 and Huh7), whereas almost no change was observed in the normal cell lines (BJ, HDF, and WI38) (FIG. 5). Accordingly, it was confirmed that the inhibition of PLRG1 expression can induce late apoptosis in a cancer cell-specific manner.

### Experimental Example 5. Confirmation of effect of PLRG1 inhibition in mesenchymal stem cells

From the results that PLRG1 is overexpressed in cancer cells or tissues and the inhibition of PLRG1 expression can induce apoptosis in a cancer cell-specific manner without affecting normal cells, it was confirmed that PLRG1 is a cancer cell-specific essential growth factor. To further support these results, the effect of PLRG1 was measured using mesenchymal stem cells with rapid cell division.

As a result, the inhibition of cell growth by the reduction of PLRG1 expression was not also observed in the mesenchymal stem cells, as was the case with other normal cells (FIG. 6). Specifically, no change by the reduction of PLRG1 expression was observed in the cell growth experiment by crystal violet staining (FIG. 6B) as well as in the observation of cell morphology under microscope (FIG. 6A).

Meanwhile, telomerase is activated in stem cells such as mesenchymal stem cells, as is the case with cancer cells. Since the effect of the reduction of PLRG1 expression in mesenchymal stem cells was not observed, for further clarification, the effect of the reduction of PLRG1 expression was measured using normal cells in which overexpression of telomerase was induced.

As a result, it was confirmed that cell growth was not affected by the inhibition of PLRG1 expression in both the BJ cells where telomerase was not expressed and the BJ-T cells where telomerase was expressed (FIGS. 7A and 7B). This confirms that telomerase is not affected by the inhibition of PLRG1 expression regardless of telomerase expression, in normal cells, unlike in cancer cells.

### Experimental Example 6. Confirmation of effect of inhibition of cancer cell growth by various PLRG1-siRNAs

For further clarification of the anticancer effect due to PLRG1 inhibition, 10 kinds of siRNAs targeting the PLRG1 gene were further prepared by varying sequences (FIG. 8A). Additionally, each siRNA was transfected into Huh7 and SNU475, and the effects of the transfection on cell growth and PLRG1 expression were measured.

As a result, it was confirmed that the PLRG1 expression was inhibited by the 10 kinds of siRNAs (siPLRG1 #1 to siRLRG1 #10) in both Huh7 and SNU475 cell lines, and accordingly, the number of viable colonies was significantly reduced (FIG. 8B). Therefore, it was confirmed that the effect of PLRG1-siRNA was not limited to those siRNAs which target particular nucleotide sequences. These results suggest that once the expression of the PLRG1 gene is inhibited it can result in the inhibition of cell growth in a cancer cell-specific manner regardless of the method.

### Experimental Example 7. Confirmation of effect of PLRG1 inhibition in various cancer cell lines

To confirm whether the cancer cell apoptosis by the inhibition of PLRG1 is limited to particular cancers such as liver cancer, *etc.* or is a phenomenon which can be generally applicable all cancer cells regardless of the origin of tissues, a further experiment was performed using various cancer cell lines.

As a result, it was confirmed that the number of viable colonies was significantly decreased in all of the lung cancer cells (A549, H460), breast cancer cells (MDA231), uterine cervix cancer cells (Hela, SiHa), osteosarcoma cells (SaOS2, U2OS), brain cancer cells (LN229), and colon cancer cells (HCT116, SW480), when the PLRG1 expression was inhibited (FIG. 9). Accordingly, it was confirmed that the cancer cell apoptosis due to the inhibition of PLRG1 expression is not limited to particular forms of cancer, but it is widely applicable to all types of cancer.

### Experimental Example 8. Preparation of IPTG-inducible sh-PLRG1 system and confirmation of inhibitory effect against cancer cell growth thereof

To further characterize the phenomenon of inhibiting the cancer cell growth by PLRG1 deficiency, the present inventors have prepared a system for the control of PLRG1 expression by IPTG-inducible sh-PLRG1. A constitutive lenti-vector containing shRNA for human PLRG1 (NM_002669) was purchased from Sigma (FIG. 10A). Oligonucleotides were synthesized so as to introduce the nucleotide sequences shPLRG1 #1 and shPLRG1 #2, which were confirmed in the constitutive lenti-vector, into the inducible lenti-vector system. Primers were annealed with reference to shPLRG1 #1 and shPLRG1 #2, and then the synthesized PCR products were digested with *Kpn*I*-Eco*RI and cloned into the LKO-3X inducible vector. The information on oligonucleotide sequences are as follows.
shPLRG1 #1-F (*Kpn*I):
   CgaGgtaCCGGCCATGATACTACAATTCGATTCTCGAGAATCGAATTGTAGTATCATG GTTTTTagaattcCG (SEQ ID NO: 23)
shPLRG1 #1-R (*Eco*RI):
   CGgaattctAAAAACCATGATACTACAATTCGATTCTCGAGAATCGAATTGTAGTATCA TGGCCGGtacCtcg (SEQ ID NO: 24)
shPLRG1 #2-F (*Kpn*I):
   CgaGgtaCCGGGACTTGGCTAGTGGCAAATTACTCGAGTAATTTGCCACTAGCCAAGT CTTTTTTGaattcCG (SEQ ID NO: 25)
shPLRG1 #2-R (*Eco*RI):
   CGgaattCAAAAAAGACTTGGCTAGTGGCAAATTACTCGAGTAATTTGCCACTAGCCA AGTCCCGGtacCtcg (SEQ ID NO: 26)

Viruses were synthesized with reference to the lenti-vectors in which the cloning was completed and transfected into the liver cancer cell lines (Huh7 and SNU475) and normal cell line (BJ). The cells were maintained for 7 days, and as a result, it was confirmed that the normal cell line was not affected by apoptosis while apoptosis was induced in the liver cancer lines by the sh-RNA target sequences of sh-PLRG1 #1 and sh-PLRG1 #2 (FIG. 10B). Viruses were synthesized with reference to the two kinds of lenti-vectors (*i.e*., sh-PLRG1 #1 and sh-PLRG1 #2), transfected into the liver cancer cell lines (Huh7 and SNU475) and normal cell line (BJ) for 2 days, and the infected cells were selected using puromycin for 5 days. Then, the cells were maintained under the treatment of 1 mM IPTG for 10 days, and as a result, it was confirmed that the normal cell line was not affected by apoptosis while apoptosis was induced in the liver cancer lines by the sh-RNA target sequences #1 and #2 (FIG. 10C). The apoptosis of the liver cancer cell lines by the sh-RNA target sequences #1 and #2 was also confirmed in another set of experiments, and in particular, the expression level of the PLRG1 was shown to be significantly reduced compared to the sequence of the sh-control group (FIG. 10D).

### Experimental Example 9. Confirmation of inhibitory effect against PLRG1 in animal model using IPTG-inducible sh-PLRG1 system

Huh7 cells (each 3 × 10⁶ cells), in which IPTG-inducible sh-PLRG1 #1 or sh-PLRG1 #2 was transfected, were injected into the right femur of each nude mouse while Huh7 cells (each 3 × 10⁶ cells), in which sh-control vector was transfected, were injected into the left femur of each nude mouse. Then, the nude mice were bred with water with or without IPTG for 27 days and the presence of tumor formation in each mouse was examined.

As a result, in the case of mice bred while feeding with IPTG-containing water, it was confirmed that tumor formation was observed in Huh7 transfected cells (*i.e*., cells transfected with sh-PLRG1 #1 or sh-PLRG1 #2), however, the tumors eventually disappeared along with the decrease in growth after a certain period of time. However, in the case of the mice bred while feeding with IPTG-free water, it was confirmed that the size of tumors continued to grow. Additionally, in the case of mice to which Huh7 cells having the sh-control group were injected, the growth continued regardless of the presence of IPTG (FIG. 11).

From these results, it was confirmed that the inhibition of PLRG1 expression does not affect normal cells such as mesenchymal stem cells, but induces apoptosis in a cancer cell-specific manner. Accordingly, it is expected that agents capable of inhibiting PLRG1 expression can be used as an anticancer agent, and additionally, the PLRG1 inhibitor can be used for cancer diagnosis, screening of anticancer agents, *etc.* by measuring the expression levels of PLRG1.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A pharmaceutical composition for preventing or treating cancer comprising a pleiotropic regulator 1 (PLRG1) inhibitor as an active ingredient.

2. The pharmaceutical composition of claim 1, wherein the PLRG1 inhibitor is an oligonucleotide inhibiting the expression of the PLRG1 mRNA, or an antibody inhibiting the activity of PLRG1 protein or an antigen-binding fragment thereof.

3. The pharmaceutical composition of claim 2, wherein the oligonucleotide inhibiting the expression of the PLRG1 mRNA is an antisense oligonucleotide, aptamer, shRNA, or siRNA, which is specific to the PLRG1 mRNA.

4. The pharmaceutical composition of claim 3, wherein the siRNA is double-stranded, consisting of any one oligonucleotide selected from SEQ ID NOS: 11 to 20 and an oligonucleotide having a complementary sequence thereof.

5. The pharmaceutical composition of claim 3, wherein the shRNA comprises the oligonucleotide sequence of SEQ ID NO: 21 or 22.

6. The pharmaceutical composition of claim 1, wherein the prevention or treatment is achieved by cancer cell-specific apoptosis.

7. The pharmaceutical composition of claim 1, wherein the cancer is cerebrospinal fluid tumor, brain tumor, head and neck cancer, lung cancer, breast cancer, thymoma, mesothelioma, esophageal cancer, pancreatic cancer, colon cancer, liver cancer, stomach cancer, kidney cancer, biliary tract cancer, kidney cancer, bladder cancer, prostate cancer, testicular cancer, germ cell tumor, ovarian cancer, uterine cervix cancer, endometrial cancer, colorectal cancer, lymphoma, acute leukemia, chronic leukemia, multiple myeloma, sarcoma, malignant melanoma, and skin cancer.

8. A method for treating cancer comprising administering a pharmaceutical composition comprising a pleiotropic regulator 1 (PLRG1) inhibitor as an active ingredient to a subject.

9. A composition for diagnosing cancer comprising an agent for measuring the expression level of pleiotropic regulator 1 (PLRG1).

10. A method for providing information for diagnosing cancer, comprising:
(a) measuring the expression level of pleiotropic regulator 1 (PLRG1) from an isolated biological sample;
(b) comparing the expression level measured in step (a) with that of PLRG1 of a sample of a normal control group; and
(c) when the expression level of PLRG1 from the isolated biological sample is greater than that of PLRG1 of a sample from the normal control group, establishing that cancer is present.

11. A method for screening agents for preventing or treating cancer, comprising:
(a) measuring the expression level of pleiotropic regulator 1 (PLRG1) from a sample of an experimental animal having cancer, which is a control group;
(b) administering a candidate material, which is expected to be able to treat cancer, to the experimental animal;
(c) measuring the expression level of PLRG1 from a sample of the experimental animal to which the candidate material was administered, which is an experimental group; and
(d) comparing the expression levels of PLRG1 measured in the experimental group and in the control group, and as a result, when the expression level measured in the experimental group is lower than that measured in the control group, establishing that the candidate material is able to be used as an agent for preventing cancer or an agent for treating cancer.

12. A method for screening agents for preventing or treating cancer, comprising:
(a) treating isolated cancer cells capable of expressing pleiotropic regulator 1 (PLRG1) with a candidate material for treating cancer;
(b) measuring the expression level of PLRG1 in the cancer cells treated with the candidate material; and
(c) when the expression level of PLRG1 measured in step (b) is lower than that of cancer cells not treated with the candidate material, establishing that the candidate material is able to be used as an agent for preventing cancer or an agent for treating cancer.

13. A method for screening candidate materials to be used as an agent for preventing or treating cancer, comprising designing an anticancer candidate material inhibiting the transcription or translation of pleiotropic regulator 1 (PLRG1) or inhibiting the activity of PLRG1 protein, based on the sequence of PLRG1 gene.
